# EUROPEAN PATENT APPLICATION

(11) **EP 1 129 716 A1**
(43) Date of publication of application: **05.09.2001**
(21) Application number: 01101434.7
(22) Date of filing: 23.01.2001
(51) Int. Cl.: A61K 35/78, A23L 1/10

(54) **Process for the production of components for alimentary integrators, dietetic, cosmetic and pharmaceutical formulations, obtained from sprouted seeds, and products so obtained**

(30) Priority: 08.02.2000 IT MI000197
(71) Applicant: Gianfranceschi, Gianluigi, 06137 Poggio delle Corti, Perugia PG (IT); Centro Studi Giuseppe Gianfranceschi, 60031 Castelplanio (IT)
(72) Inventor: Gianfranceschi, Gianluigi, 06137 Poggio delle Corti, Perugia PG (IT)
(74) Representative: Trupiano, Federica

(57) **Abstract**

Process for obtaining a product having an activity of regulation of cell, tissue and organ metabolism, employable as a component of formulations for alimentary integration, in the dietetic, cosmetic, pharmacological fields and the like, obtained from sprouted seeds, comprising the following steps: causing the sprout of vegetable seeds of cereals and/or legumes on a support constituted of a "soft gel", such as agar gel, by spraying with suitable dosed water, and keeping the temperature between 15°C and 30°C until the sprouts reach a height of 3-**5** (7?) cm; submitting the material constituted of the support and the sprouted seeds to dehydration, keeping the temperature between 15°C and 25°C, until there is obtained an amount of residual water in the material lower than 12% by weight; breaking and detaching the sprouts and the seed roots from the seeds and the dehydrated support in such a manner as not to shatter the seeds; and separating by sieving the broken sprouts and roots from the seeds and the dehydrated support, keeping the so obtained support in a humidity-free atmosphere.

## Description

The present invention relates to a process for the production of components for alimentary integrators and products employable in the dietetic, cosmetic, pharmaceutical fields and the like, based on sprouts of cereal and/or legume seeds. The invention relates also to alimentary integrators and various products so obtained and the galenic formulations containing such products and utilizable in the dietetic, cosmetic, pharmaceutical fields with special reference to various dysmetabolisms, also associated to senescence, immunity alteration conditions, etc.

As is known (G.L. Gianfranceschi et al., Nature 262:622, 1976;

D. Amici et al., Mechanism of Ageing and Development 23:215, 1983; G. Lugaro et al., Biochim. Biophys. Acta 950:420, 1988; G.L. Gianfranceschi et al., Peptides 15:7, 1994), some extracts of animal organs and liquids contain peptide-based, low-molecular-weight, sometimes phosphorylated substances, that can exercise either an effect of reactivation of cell, organ or systemic metabolism, or *in vitro* inhibition of DNA transcription and the growth of cancer cells.

The utilization of such products is limited by the difficulty of enriching and isolating active molecules from animal sources, while their chemical synthesis is extremely complex (especially for the part connected to phosphorylation) and very expensive economically. It must also be added that the aforementioned pharmacological activities can be brought back to one only or a restricted pool of the above peptides, the effect being likely to be associated to a synergistic action of the various components.

Object of this invention is to realize a process for the production of products having an activity of regulation of the cell, tissue and organ metabolism, employable as components for alimentary integration, and more generally products employable in dietetic, cosmetic, pharmaceutical fields and the like, with a high content of highly energetic organic phosphates, and in particular with an organic phosphate/inorganic phosphates ratio greater than 1.

Another object of the invention is to realize a process for the production of products for integrators and dietetic, cosmetic and pharmaceutical products, generally essentially natural, with high concentrations of molecules having a high **oxidation** (antioxidation?) power and practically free from substances that can induce intolerance phenomena.

Another object of the invention is to provide alimentary integrators and products employable in dietetic, cosmetic, pharmaceutical fields and the like so obtained.

These and still other objects and related advantages that will be evident thanks to the following description are achieved by a process that allows to obtain a product employable as a component for alimentary integration in dietetic, cosmetic, pharmacological fields and the like, which process, according to the present invention, comprises the following steps:
- causing the sprout of vegetable seeds on a support constituted of a "soft gel" by spraying with suitably dosed water, and keeping the temperature between 15°C and 30°C until the sprouts reach a height of 3-7 cm;
- submitting the material constituted of the support and the sprouted seeds to dehydration, keeping the temperature between 15°C and 25°C, until there is obtained an amount of residual water in the material lower than 12% by weight;
- breaking and detaching the sprouts and the roots present in said material from said seed and said dehydrated support in such a manner as not to shatter but to keep whole said seeds;
- separating by sieving the broken seeds and roots from the seeds and the dehydrated support,
- grinding said separated sprouts and roots in a humidity-free atmosphere, so as to prevent rehydration phenomena, obtaining in this way said product employable as alimentary integrator, in dietetic, cosmetic pharmacological fields and the like.

More particularly said vegetable seeds are constituted of cereal and/or legume seeds.

Besides, preferably, said "soft gel" is constituted of low concentration agar gel of the order of 0,8-1,5%.

As is known, agar is a mucilaginous substance, generally extracted from algae, soluble in hot water but insoluble in cold water, and that can absorb water up two twenty times its weight. Substantially, it is a galactose-based polymer.

Surprisingly, it has been found that the contents in substances capable to exercise both an effect of cell, organ or systemic metabolism reactivation, and to prevent DNA transcription and the growth of cancer cells is markedly high in vegetable sprouts obtained in suitable growth conditions, as described above, especially in the first 3-5 days of development. Growth conditions controlled in the time, that lead to sprouts having a comparable height, an appropriate technology of isolation of the sprouts from the seed and a suitable drying and pulverization process allow to obtain highly active and reproducible products, useful as a base for alimentary, dietetic, cosmetic and pharmaceutical formulations.

Provided that they have been obtained in compliance with the above described preparation conditions, such products show, besides the aforementioned biologic activities, a very high contents of highly energetic organic phosphates (with an organic phosphates / inorganic phosphates ratio greater than 1 and a very high anti-oxidant capacity (anti-free radical activity). Recently, many authors (Y. Niwa et al., International Journal of Tissue Reactions 20:63, 1998; H. Hocman, Comparative Biochemistry & Physiology-B 93:201, 1989; M.J. Rhodes et al., European Journal of Cancer Prevention 6:518) have stressed that such anti-radical activity plays a fundamental role in the reduction of cell, tissue and organ damages, as it is able to neutralize highly reactive chemical species produced in the animal organism by dysmetabolic conditions associated to various pathologies or senescence. Such characteristics cause the products of the present invention to be still more interesting for the application purposes mentioned.

It is known that there are already available various products obtined from wheat (and other cereals), which are essentially referable to two categories:
a) products obtained by the upper part (about 10 cm) of wheat seedlings of 20 cm of height and grown for 6-7 months in localities having a very cold winter climate; they contain especially vitamins, vegetable fibers and mineral substances.;
b) products constituted of oil obtained from wheat germ, i.e. the embryos of non sprouted seeds; they are rich in vitamin E and oligoelements.

The products obtained from powder of vegetable seed sprouts are sharply different from these two categories of products. In fact, the products of the first category do not contain the molecular elements that characterize seed embryo. Those of the second category, based on wheat germ, contain potentially the same molecular and enzymatic complex, but in an inactive form: in fact, the seed embryo apparatuses undergo an activation and a progressive differentiation just when they are activated by the sprouting process. Particularly marked is the activation of protein kinases that catalyze the phosphorylation of proteins and numerous other cellular substrates.

In the following there is reported the phosphor contents in wheat germ, wheat sprout and wheat seedling in the dehydrated state; The values are expressed in mg for each 100 g of product:

| | |
|---|---|
| wheat germ | 840 ± 130 |
| wheat sprout | 4800 ± 800 |
| wheat seedling | 530 ± 90 |

As can be observed from such values, the total phosphate content of sprout powder obtained in the conditions of the present invention is about 5-6 times higher than the germ, and 9-10 times higher than the seedling. Besides, if one determines the percentage of highly energetic organic phosphates (ester bonds and especially acilic bonds), the difference is still higher: in fact, the organic phosphates contained in the sprout are about 9-10 times higher with respect to the germ and 14-15 times higher with respect to the seedling.

The total phosphorylation activity of the wheat germ reaches its peak after 3-4 sprouting days.

| Sprouting days | Sprout length (mm) | Phosphorylation activity (%) |
|---|---|---|
| 1 | 0.5 - 1 | 20 |
| 2 | 1 - 3 | 50 |
| 3 | 5 - 15 | 100 |
| 4 | 20 - 40 | 100 |
| 5 | 40 - 70 | 100 |
| 6 | 60 - 80 | 70 |
| 7 | 80 - 100 | 30 |

(100% is the peak of the activity).

Usually, seed sprouting is carried out in laboratories on filter paper, but to produce high amounts this system has serious drawbacks, as sprouts root in the inside of the wet paper, and therefore at the end of sprouting it is necessary to detach by hand the sprouted seeds from the paper.

The sprouting of cereal seeds is practiced since ancient times, on soil, wet paper and also wet fabric. The utilization of agar gel as a substrate whereon vegetable seeds are caused to sprout has started only a few years ago; according to the process of the present invention, low concentration agar (0,8-1,5%) is used, which give rise to said "soft gel".

Substances of biologic interest, such as vitamins, mineral substances and various oligoelements may be added to the water sprayed to cause sprouting and/or the water used to realize said soft gel, and said substances are then incorporated by the seed during the sprouting step.

According to the process of the invention, seeds, for instance wheat seeds, are washed and then hydrated in water for 12-24 h; dehydration water may be enriched with substances that can be incorporated by wheat grains, for instance vitamins, mineral substances and various oligoelements. The so hydrated wheat, drained from excess water, is deposited on a layer of agar gel having a thickness of 2-3 mm. The optimal concentration of agar in the gel is comprised between 0.8 and 1.5%; the optimal amount of wheat to be layered on the gel to cause sprouting is of 20-30 g/100 cm². It is possible to add substances of biologic interest also in the water where agar is dissolved to produce the gel. Agar is dissolved at a temperature of about 90°C; during the cooling step, before gelling, said substances of biologic interest may be added to the solution, which substances can be in this way assimilated by wheat which during sprouting roots in the gel.

Wheat layered on gel is sprayed with water: about 10 ml water per each 100 cm² of surface; the trays containing the wheat to be sprouted are placed in a room having a temperature comprised between 18 and 20 °C; after 24 h, the operation of water spraying is repeated as above; after 4-5 days of sprouting, when sprouts reach a height of 3-5 cm, the gel carrying the sprouted wheat is dismembered into pieces of 20-40 cm², that are then placed on a frame provided with a plastic net, and invested with a continuous flow of dehumidified air, at a temperature of 15-25°C, to obtain the dehydration of the material.

After 48-72 hours, when the percentage of residual water is lower than 12% the total weight, the sprouted wheat is placed in a cutter having a capacity of 5-10 liters, provided with only slightly sharp and therefore poorly cutting blades. Starting from the minimum, the cutter is brought to the maximum speed and so maintained for about 20 seconds. After an interruption of 20 seconds, the operation is repeated. However, such conditions must be so determined as to achieve the result of detaching the sprout from the seed and of partly breaking it without shattering the seed.

The so obtained material is sieved with a sieve having a porosity of 2-2,5 mm x 2-2,5 mm, so that the fragments of dehydrated sprouts (and roots) go through the pores, while the wheat grain with the fragments of dehydrated agar gel (that have the appearance of a transparent film) remain on the sieve; the so isolated fragments of sprouts (and roots) are then finely ground with a cutter having a capacity of 0.5-1 liter, provided with very sharp blades operating 10 homogenization steps of 15 seconds each at the maximum speed, and alternating each step with a 15 sec pause to prevent the material from heating. The so obtained wheat sprout powder is preserved, preferably in hermetically sealed vacuum vessels, to prevent the partial rehydration of the product.

The dehydration carried out according to the present invention with an air flow at temperatures not higher than 25°C ensures the preservation of all the substances having a biological value; in order to obtain the complete dehydration of the seeds sprouted at such temperatures, air is caused to pass through a dehumidifier before investing the sprouted seeds.

The separation of the sprouts from the seeds after a complete dehydration is a technological point of great importance, as the substances that are of interest from the biological point of view are contained in the sprout, while the seed contains mainly amid. Besides, some substances present in the seed cuticle may induce intolerance phenomena in some subjects. Treating the sprouted and dehydrated seeds with a cutter for a very short time (twice for 15-20 sec) taking care to use only slightly sharp blades, allows to achieve the result of detaching and breaking the sprouts without shattering the seed.

Said product employable as an alimentary integrator and for formulations in dietetic, cosmetic, pharmacological fields and the like comprises:

| | |
|---|---|
| Water | max. 12% by weight |
| Ashes | 2.5 - 4% by weight |
| Proteins | 10.0- 14.5% by weight |
| Fats | 2.0 - 3,0 by weight |
| Soluble sugars | 4,0 - 6,5% by weight |
| Amid | 58.0 - 72.0% by weight |
| Fiber | 3.0-4.5% by weight |
| Potassium | 450-750 mg/100 g |
| Sodium | 45-70 by weight |
| Calcium | 120-200 by weight |
| Magnesium | 60-90 by weight |
| Iron | 0,02-0,05 by weight |
| Zinc | 10-16 by weight |
| Copper | 0,35-0,55 by weight |
| Phosphorous | 4000-5600 by weight |
| Antioxidant substances (such as glycosides, in particular flavonoids, polyphenols, substances with thio groups) | 2500-4000 by weight |
| Organic phosphates/inorganic phosphates ratio > 1 | |

Another element of importance from the nutritional and pharmacological point of view is the fact that about 40% of fats present is constituted by linolenic acid.

The product obtained according to the present invention has many application possibilities, in particular thanks to the high contents of organic phosphates that, as is known, are very energetic (ester bonds and acilic bonds), and the high concentrations of molecules having a high antioxidant power. The product can be used in the fields of alimentary integrators, either alone or asscociated to other substances. Besides, it can be used as a material wherefrom classes of alimentary and pharmacological interest substances can be extracted as a base of higher- concentration more-targeted products; for instance, there may be obtained:
- the fraction of organic phosphates to be used in malnutrition, weakness, convalescence cases and to improve memory,
- the fraction of antioxidant substances to be used as a prevention and treatment of disorders associated to aging: cataract, blood vessel sclerosis, reduction in immune response, constipation, etc.

### Example 1

1000 g wheat (*Triticum durum)* are washed, mixed with 2-3 liter water and so kept at ambient temperature, for instance for 12-14 hours;
in the water wherein wheat is caused to hydrate for 12-24 h, there may be introduced substances to be absorbed by the seed, such as for instance:
- vitamins: e.g., Vitamin C at a concentration of 1-10 mM;
- mineral salts: e.g., selenium chloride at a concentration of 1-10 mM;
after 12-24 hours, the so hydrated wheat is drained from excess water and deposited on a layer of agar gel having a thickness of 2-3 mm, prepared on circular paper trays of a diameter of 30 cm or rectangular plastic trays of 30 x 45 cm.

For each circular tray, 140-210 ml of 0.8-1.5% agar solution to be gelled are placed; for each rectangular tray, 270-400 ml are required.

In the agar solution substances may be added that are incorporated by the seed, at the same concentrations as specified above. To cause the sprouting of 1000 g wheat, 5-7 circular trays or 3-4 rectangular trays are required.

At the end of the preparation, from 1000 g wheat there are obtained 250-350 g of dehydrated sprouts and therefore of sprout powder.

### Example 2

As concerns the high antioxidant activity observed in sprouts of vegetable seeds, aqueous extracts of the wheat sprout powder (10 g sprout powder are resuspended in 200 ml water; the suspension is homogenized with a high speed Blendor homogenizer 4 times for 30 seconds, alternating each step with a 30 sec pause; the homogenate is centrifuged at 10000 x g for 30 minutes and the supernatant is recovered) show the following activities:
1st - total antioxidant activity

The extract corresponding to 0.1 mg sprout powder has a marked reaction with phosphomolybdic acid, which reacts aspecifically with any antioxidant substance. The extract is submitted to thin layer chromatography utilizing as eluent silica gel and propanol/water slides (70:30, v/v). After the chromatography, the slide is dried, sprayed with 10% phosphomolybdic acid in ethanol and heated on a plate: the antioxidant substances cause the appearance of blue stains.
2^{nd} - anti-free radical activity

The extract corresponding to 10 mg sprout powder can capture *in vitro* in 2 ml almost all the superoxide radicals by the xanthine-xanthine oxidase system, utilizing 0.9 U/ml xanthine oxidase and micromolecular xanthine 50 (R. Gabbianelli et al., Free Radical Biology & Medicine 23:278, 1997). Experiments carried out on wheat germ and wheat seedling show that the main reducing-activity fraction isolated from wheat sprout lacks in the germ, while the seedling contains 15-20 times less.

## Claims

1. A process for obtaining a product having an activity of regulation of cellular, tissue and organ metabolism, employable as a component of formulations for alimentary integration, and for dietetic, cosmetic, pharmacological formulations and the like, derived from sprouted seeds, **characterized in that** it comprises the following steps:
- causing the sprout of said vegetable seeds of cereals and/or legumes on a support constituted of a "soft gel" by spraying with suitable dosed water, and keeping the temperature between 15°C and 30°C until the sprouts reach a height of 3-7 cm;
- submitting the material constituted of said support and said sprouted seeds to dehydration, keeping the temperature between 15°C and 25°C, until there is obtained an amount of residual water in said material lower than 12% by weight;
- breaking and detaching said sprouts and the roots present in said material from said seeds and said dehydrated support in such a manner as not to shatter but to keep said seeds whole;
- separating by sieving or other suitable means said broken sprouts and said roots from said seeds and said dehydrated support:
- grinding said separated sprouts and said roots in a humidity-free atmosphere, so as to prevent rehydration phenomena, obtaining in this way a product employable as alimentary integrator, in the dietetic, cosmetic, pharmacological fields and the like.

2. The process according to claim 1, **characterized in that** said vegetable seeds are cereal seeds.

3. The process according to claim 1, **characterized in that** said seeds of vegetable species are constituted of wheat seeds (*Triticum*).

4. The process according to claim 1, **characterized in that** said "soft gel" is constituted of agar gel at low concentrations, in the order of 0.8-1.5%.

5. The process according to claim 1, **characterized in that** substances of biologic interest such as vitamins, various mineral substances and oligoelements are added to the water sprayed to cause the sprouting and/or the water employed to realize said soft gel, said substances of biologic interest being then incorporated by wheat during the sprouting step.

6. The process according to claim 1, **characterized in that** said dehydration is carried out by means of a flow of dehumidified water at a temperature of 15-25°C.

7. The process according to claim 1, **characterized in that** said sprouts and roots are detached from said seeds and said dehydrated support by a cutter provided with only slightly sharp and therefore poorly cutting and batch-working blades.

8. A product employable for alimentary use, in dietetic, cosmetic and pharmacological fields and the like, realized according to the process of claim 1, **characterized in that** it comprises:
| | |
|---|---|
| Water | max. 12% by weight |
| Ashes | 2.5 - 4% by weight |
| Proteins | 10.0- 14.5% by weight |
| Fats | 2.0 - 3,0 by weight |
| Soluble sugars | 4,0 - 6,5% by weight |
| Amid | 58.0 - 72.0% by weight |
| Fiber | 3.0-4.5% by weight |
| Cations such as K and/or Na and/or Ca | |
| and/or Mg and/or Fe | |
| and/or Zn and/or Cu | 500-1000 mg/ |
| and/or, etc. | 100 g |
| Phosphorous | 4000-5600 mg/ |
| Antioxidant substances (such as glycosides, in particular flavonoids, polyphenols, substances with thio groups) | 2500-4000 mg/ |
| Organic phosphate/inorganic phosphates ratio >1 | |

9. The process according to claim 8, **characterized in that** about 40% fats are represented by linolenic acid.

10. The alimentary, dietetic, cosmetic, pharmaceutical use of the products obtained according to claims 1-8, **characterized in that** they have a markedly constant and reproducible composition, and an activity of regulation of the cell, tissue and organ metabolism, by means of incorporation in suitable formulations (oral, topical, etc.).
